# EUROPEAN PATENT SPECIFICATION

(11) **EP 1 239 875 B1**
(45) Date of publication and mention of the grant of the patent: **01.03.2006**
(21) Application number: 00987927.1
(22) Date of filing: 20.12.2000
(51) Int. Cl.: A61K 39/085

(54) **USE OF STAPHYLOCOCCAL BACTERIA TO TREAT IRRITABLE BOWEL SYNDROME**
VERWENDUNG STAPHYLOKOKKEN-BAKTERIEN ZUR BEHANDLUNG DES KOLON IRRITABILE
UTILISATION D'UNE BACTERIE STAPHYLOCOCQUE POUR LE TRAITEMENT DU SYNDROME DU COLON IRRITABLE

(30) Priority: 21.12.1999 SE 9904719
(43) Date of publication of application: 18.09.2002
(73) Proprietor: Gottfriesmottagningen AB, 431 80 Mölndal (SE)
(72) Inventor: GOTTFRIES, Carl-Gerhard, S-414 75 Göteborg (SE); REGLAND, Björn, S-431 35 Mölndal (SE)
(74) Representative: Cederbom, Hans Erik August
(86) International application number: PCT/SE2000/002592
(87) International publication number: WO 2001/045736

(56) References cited:
- WO-A1-90/01335
- WO-A1-96/25155
- WO-A1-98/29133
- ANDERSSON M. ET AL EUR. J. PAIN vol. 2, 1998, pages 133 - 142
- STAPHYPAN BERNA (FACHINFORMATION DES ARZNEIMITTEL-KOMPENDIUM DER SCHWEIZ)

## Description

The present invention relates to a procedure for the use of staphylococcal bacteria or a product derived therefrom.

Irritable Bowel Syndrome (IBS) is a bowel disease with variable symptoms, which may be referred to in Swedish as the equivalent of "irritable large intestine". There are no biological markers for the disease. Two systems of classification based on the symptoms have been drawn up to define the disease. One is known as the "Manning criteria" and the other as the "Rome criteria".

In Manning, A.P., Thompson, W.G., Heaton, K.W., Morris, A.F. (1978), "Towards Positive Diagnosis of the Irritable Bowel", Br. Med. J., 2:653-654, four symptoms are identified which, in a significant fashion, distinguish patients with IBS from patients with some other known organic disease; these are tension and swelling in the abdomen, pain alleviation in conjunction with bowel activity, more frequent bowel motions in conjunction with abdominal pains and looser bowel motions in conjunction with abdominal pains. In addition to these, it was also stated that two symptoms are also often encountered, namely mucous-like motions and a sense of incomplete evacuation of the bowel.

The Rome criteria, to the extent that these have been applied, define IBS as a period of abdominal pain or abdominal discomfort of at least three months' duration, which is alleviated in conjunction with bowel motion, or changes in the frequency or consistency of the bowel motion. Two or more of the following symptoms must exist for a fairly short period: changed frequency of bowel motion and/or changed consistency, bowel motion in the form of mucous, flatulence and/or a feeling of tension in the abdomen. This is described in the following works:
□ Manning, A.P., Thompson, W.G., Heaton, K.W., Morris, A.F. (1978), "Towards Positive Diagnosis of the Irritable Bowel", Br. Med. J., 2:653-654.
□ Drossman, D.A., Thompson, W.G. (1992), "The Irritable Bowel Syndrome: Review and a Graduated Multicompmonent Treatment Approach", Ann. Intern. Med., 116:1009-1016.
□ Thompson, W.G. (1993), "Irritable Bowel Syndrome: Pathogenesis and Management", The Lancet, 341:1569-1672.
□ Thompson, W.G., Credd, F., Drossman, D.A., Heaton, K.W., Mazzaca, G. (1992), "Functional Bowel Disease and Functional Abdominal Pain", Gastroenterol. Int., 2:75-91.
□ Hahn, B.A., Saunders, W.B., Maier, W.C. (1997), "Differences between individuals with self-reported irritable bowel syndrome (IBS) and IBS-like symptoms", Diagnostic Diseases and Sciences, 42:2585-2590.

The prevalence of IBS is reported to be 9.4% in a random sample of 5 430 adult US citizens investigated in 1990. In another study, the prevalence was claimed to be 7.2% if the Rome criteria were applied, and 17.0% if the Manning criteria were applied. Regardless of the method used, findings have been made which indicate the existence of a large group of individuals whose disease has not been diagnosed as IBS, but who exhibit symptoms similar to those encountered in these syndromes. A summary can be obtained by studying the article by Hahn, B.A., Saunders, W.B., Maier, W.C. (1997), "Differences between individuals with self-reported irritable bowel syndrome (IBS) and IBS-like symptoms", Diagnostic Diseases and Sciences, 42:2585-2590.

One of the conclusions reached in the aforementioned article by Hahn and his colleagues is that 17.6 million adult US citizens suffer from IBS. On the other hand, only 6.4 million have been diagnosed as having IBS.

There is no known specific treatment for IBS.

WO 98/29133 discloses the use of the staphylococcal vaccine Staphypan Berna for the manufacture of pharmaceutical preparation for treatment of fibromyalgia and chronic fatigue syndrome (CFS). Irritable bowel syndrome (IBS) is mentioned as one of several symptoms related to fibromyalgia (see page 2, lines 11-12).

Document Andersson, M. et al (1998) Eur. J. Pain 2: 133-142 discloses the use of the staphylococcus toxoid vaccine on pain and fatigue in patient with fibromyalgia/chronic fatigue syndrome. Irritable bowels syndrome (IBS) is also mentioned as one of several symptoms related to fibromyalgia (see page 1 column 2 lines 15-16).

The told prior art does not, however, give any information about the relationship between irritable bowels syndrome (IBS) and staphylococcal infections. Consequently, none of the told documents do disclose that the disorder can be treated by an immune modulating treatment in the form of staphylococcus preparation.

It is an object of the present invention to make available a means for the therapeutic treatment of IBS.

The aforementioned object is achieved by means of the use of a staphylococcal toxoid vaccine for the production of a pharmaceutical preparation for the treatment of irritable bowel syndrome (IBS).

The invention is described below as a number of preferred illustrative embodiments, in conjunction with which reference is made to a Figure in which an example of treatment with means in accordance with the invention is shown.

In accordance with the present invention, the means for the aforementioned therapeutic treatment of IBS has been produced by the use of a staphylococcal vaccine, Staphypan Berna, which is preferably injected subcutaneously. This favourable effect, which could be established in a clinical study, is presumably attributable to the stimulation of immune activity in the patient and is not perceived as a traditional form of vaccination. Treatment preferably takes the form of a series of injections of Staphypan Berna at an increasing dose over a period of time, for example six months, after which treatment is maintained with one injection per month.

This can be developed in accordance with what is indicated in the Patent Claims.

### Description of earlier studies:

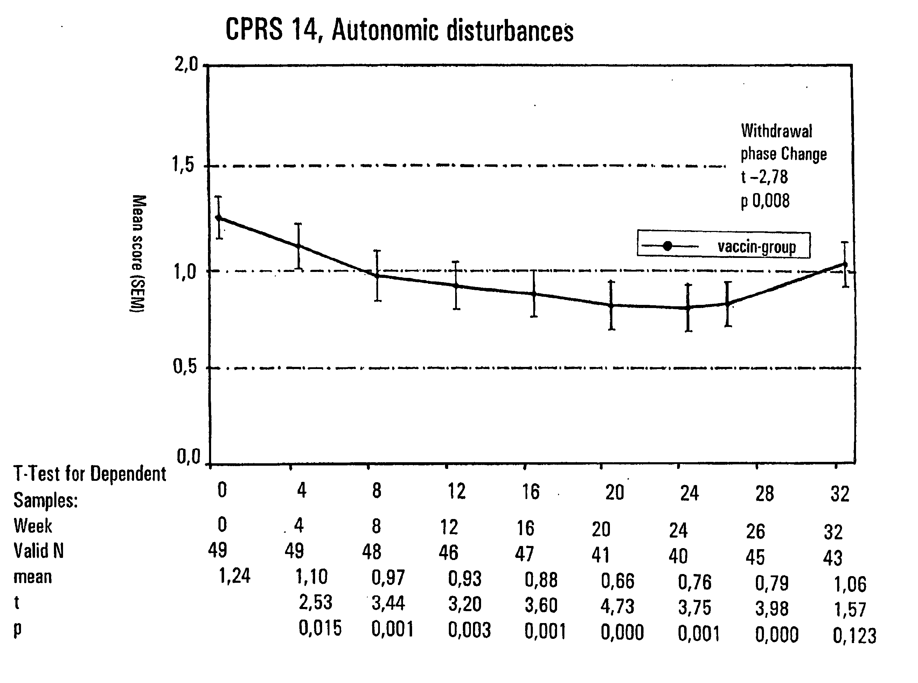

Confidential treatment trials with a staphylococcal vaccine, Staphypan Berna, have been conducted in the Department of Psychiatry at the Sahlgrenska University Hospital in Mölndal, Sweden. One surprising finding to emerge from these treatment studies, which in part were of the controlled type and were carried out in accordance with the double-blind method, was that a sub-group of patients who were suffering from IBS showed an improvement in relation to this disease. In the clinical studies, the patients were assessed on the basis of the Comprehensive Psychopathological Rating Scale (CPRS) assessment scale. This assessment scale includes a sub-scale referred to as "Autonomic disturbances", which is defined as follows: "Representing description of palpations, breathing difficulties, dizziness, increasing sweating, cold hands and feet and dry mouth, indigestion, diarrhoea and frequent nicturition". In connection with assessments made using this sub-scale, a clear improvement could be noted in the symptoms associated with the IBS syndrome. In the group of patients who had been treated with active Staphypan vaccine, a significant improvement could already be observed after four weeks' treatment when the patients' base values were compared with the estimated values after four weeks. The improvement continued to increase until week 26, when the treatment was discontinued (see the Figure).

### Procedure:

Staphypan Berna is an anti-infectious and antitoxic staphylococcal vaccine, which is currently produced by the Swiss Serum and Vaccine Institute in Berne, Switzerland.

The patients received the vaccine in an increasing dose over a period of eight weeks. A dose of 0.1 ml was given in the first week, and this was increased successively to 1.0 ml. After eight to ten weeks, the patients received 1 ml of the vaccine per week for a further four weeks, after which they received 1 ml of the vaccine once a month. The vaccine was administered by a nurse under the skin (subcutaneously) in the gluteal region (buttock).

### Laboratory examinations.

The patients who were included in the clinical trial and were suffering from IBS were also subjected to routine laboratory examinations. No indications of other diseases or abnormal laboratory values were found.

### Follow-up

Those patients who were included in the clinical trial and were treated with Staphypan Berna ceased the treatment with Staphypan Berna after 24 weeks. It was observed that, after two months, there was a significant impairment in the IBS symptoms in the group that had received active treatment with Staphypan Berna (see the Figure). The vaccine was administered under blind conditions. A total of around 100 patients were studied and followed up at a later date, when it was observed that the improvement was maintained if the treatment was continued.

The invention is not restricted to the above description, however, but can be varied within the scope of the Patent Claims without departing from the idea of invention.

## Claims

1. Use of a staphylococcal toxoid vaccine for the production of a pharmaceutical preparation for the treatment of irritable bowel syndrome.

2. The use of claim 1 **characterized in that** the preparation is to be administrated subcutaneously.

3. The use of claims 1 or 2 **characterized in that** the preparation is administrated initially in an increasing dose and thereafter at regular intervals.

## Patentansprüche

1. Verwendung eines Staphylokokken Toxoid-Impfstoffes für die Herstellung eines pharmazeutischen Präparats zur Behandlung des Reizdarmsyndroms.

2. Verwendung nach Anspruch 1, **dadurch gekennzeichnet, dass** das Präparat subkutan zu verabreichen ist.

3. Verwendung nach Anspruch 1 oder 2, **dadurch gekennzeichnet, dass** das Präparat anfänglich in einer ansteigenden Dosis und danach in gleichmäßigen Intervallen verabreicht wird.

## Revendications

1. Utilisation d'un vaccin toxoïde staphylococcique pour la fabrication d'une préparation pharmaceutique pour le traitement du syndrome du colon irritable.

2. Utilisation selon la revendication 1, **caractérisé en ce que** la préparation doit être administrée par voie sous-cutanée.

3. Utilisation selon les revendications 1 ou 2, **caractérisée en ce que** la préparation est administrée initialement à doses croissantes et ensuite à intervalles réguliers.
